# FASCICULE DE BREVET EUROPEEN

(11) **EP 1 850 900 B1**
(45) Date de publication et mention de la délivrance du brevet: **11.05.2016**
(21) Numéro de dépôt: 06709250.2
(22) Date de dépôt: 06.02.2006
(51) Int. Cl.: A61M 16/04

(54) **SONDE RESPIRATOIRE**
ATEMTUBUS
RESPIRATORY TUBE

(30) Priorité: 23.02.2005 FR 0501817
(43) Date de publication de la demande: 07.11.2007
(73) Titulaire: Boussignac, Georges, 92160 Antony (FR)
(72) Inventeur: Boussignac, Georges, 92160 Antony (FR)
(74) Mandataire: Coralis Harle
(86) Numéro de dépôt international: PCT/FR2006/000261
(87) Numéro de publication internationale: WO 2006/090043

(56) Documents cités:
- EP-A- 0 106 780
- EP-A- 0 640 355
- EP-A- 0 712 638
- WO-A-99/66975
- US-A- 3 481 339
- US-A- 4 091 816
- US-A- 5 819 723
- US-A1- 2002 029 778

## Description

La présente invention concerne une sonde respiratoire comportant au moins un tube souple incompressible, par exemple en une matière synthétique telle qu'un chlorure de polyvinyle, un polyéthylène ou analogue, ladite sonde étant apte à être introduite par voie buccale ou par voie nasale, dans la trachée d'un patient, dont on veut faciliter ou assister la respiration.

On connaît par les documents US 3 481 339 A, EP 0 712 638 A, EP 0 640 355A, WO99/66975A et EP0 106 780 A divers types de sondes à ballonnets. Én particulier, le document US 4 091 816 A divulgue une sonde à deux ballonnets gonflés en série dont un est étendu en longueur. On connaît également par US 2002/029778 A1 et US 5 819 723 A d'autres types de sondes.

Quoique non exclusivement, la sonde conforme à la présente invention est tout particulièrement appropriée à être réalisée en petite taille pour assister la respiration des enfants, notamment des nouveaux-nés et des prématurés.

On sait que, lorsqu'une telle sonde est en place dans les voies respiratoires supérieures d'un patient, ledit tube souple incompressible blesse, par frottement, les muqueuses de ces voies respiratoires, de sorte que la sonde devient rapidement douloureuse pour le patient.

La présente invention a pour objet notamment de remédier à cet inconvénient.

A cette fin, selon l'invention, la sonde respiratoire comportant au moins un tube souple incompressible, apte à être introduit par voie buccale ou nasale dans la trachée d'un patient, est remarquable en ce que ledit tube est entouré, au moins sur la plus grande partie de sa longueur, par au moins une gaine souple gonflable apte à isoler, dudit tube souple, les muqueuses des voies respiratoires dudit patient.

Ladite gaine souple gonflable peut être de même nature que les ballonnets gonflables que l'on utilise dans certaines sondes médicales pour les maintenir en place dans un canal morphologique. Cependant, dans la présente invention, ladite gaine souple n'a qu'un rôle de protection des muqueuses et ne s'applique pas forcément sous pression contre ces dernières, comme le font lesdits ballonnets connus.

On remarquera que, du fait des blessures qu'elles occasionnent, les sondes connues sont toujours choisies aussi courtes que possible, leur extrémité distale se trouvant généralement à l'entrée de la trachée, juste en aval du larynx. Aussi, lorsqu'on utilise ces sondes connues pour injecter un gaz d'assistance respiratoire dans les poumons du patient, il existe un volume mort dans la trachée entre l'extrémité distale des sondes et les bronches, une partie dudit gaz d'assistance respiratoire ne pouvant pénétrer dans les poumons et étant gaspillée.

En revanche, dans la sonde conforme à l'invention, la longueur peut être telle que l'extrémité distale de ladite sonde soit logée dans la carène, puisqu'aucune blessure n'est à redouter. Le gaz respiratoire est alors délivré directement aux bronches, sans espace mort, ni gaspillage.

Par ailleurs, dans les sondes connues, il est usuel d'injecter un gaz respiratoire de ventilation à travers des canaux de faible diamètre pratiqués dans l'épaisseur de la paroi de la sonde. Bien entendu, ces sondes connues non seulement présentent, pour le gaz de ventilation, les mêmes inconvénients que pour le gaz d'assistance respiratoire (volume mort, gaspillage), mais encore nécessitent que ledit gaz de ventilation soit sous pression élevée pour traverser lesdits canaux. Il est donc nécessaire d'avoir à disposition des sources d'un tel gaz sous pression, généralement onéreuses et délicates à utiliser, et de prendre des précautions complexes pour éviter la blessure des muqueuses des patients par des jets de gaz sous pression.

La présente invention permet d'éviter ces inconvénients supplémentaires, en utilisant ladite gaine de protection gonflable pour injecter à basse pression le gaz de ventilation. A cet effet, ladite gaine de protection est gonflée avec le gaz de ventilation et on prévoit, du côté distal de la sonde et à l'intérieur de ladite gaine, au moins un passage traversant ledit tube, afin que le gaz de ventilation gonflant la gaine puisse passer de celle-ci à l'intérieur dudit tube et, de là, dans la trachée dudit patient.

Plus précisément, l'objet de l'invention se rapporte à une sonde respiratoire telle que revendiquée en 1.

éventuellement, notamment lorsque l'étanchéité est nécessaire entre la sonde et la trachée pour éviter aux sucs gastriques de pénétrer dans les poumons du patient, la sonde respiratoire conforme à la présente invention peut :
- soit comporter une gaine souple gonflable supplémentaire entourant de façon étanche ladite gaine de protection souple gonflable ;
- soit être telle que ladite gaine de protection souple gonflable dégage l'extrémité distale dudit tube souple incompressible et que ladite extrémité distale dudit tube porte un ballonnet gonflable.

Les figures du dessin annexé feront bien comprendre comment l'invention peut être réalisée. Sur ces figures, des références identiques 15 désignent des éléments semblables.

La figure 1 est une coupe longitudinale schématique d'un exemple de réalisation de la sonde respiratoire conforme à la présente invention, la gaine souple de protection étant à l'état dégonflé.

La figure 2 est une vue semblable à celle de la figure 1, ladite gaine souple de protection étant à l'état gonflé.

La figure 3: illustre schématiquement la sonde des figures 1 et 2, mise en place dans les.voies respiratoires supérieures d'un patient.

Les figures 4 et 5 montrent à l'état gonflé, en coupe longitudinale schématique, deux autres exemples de réalisation de la sonde respiratoire conforme à la présente invention.

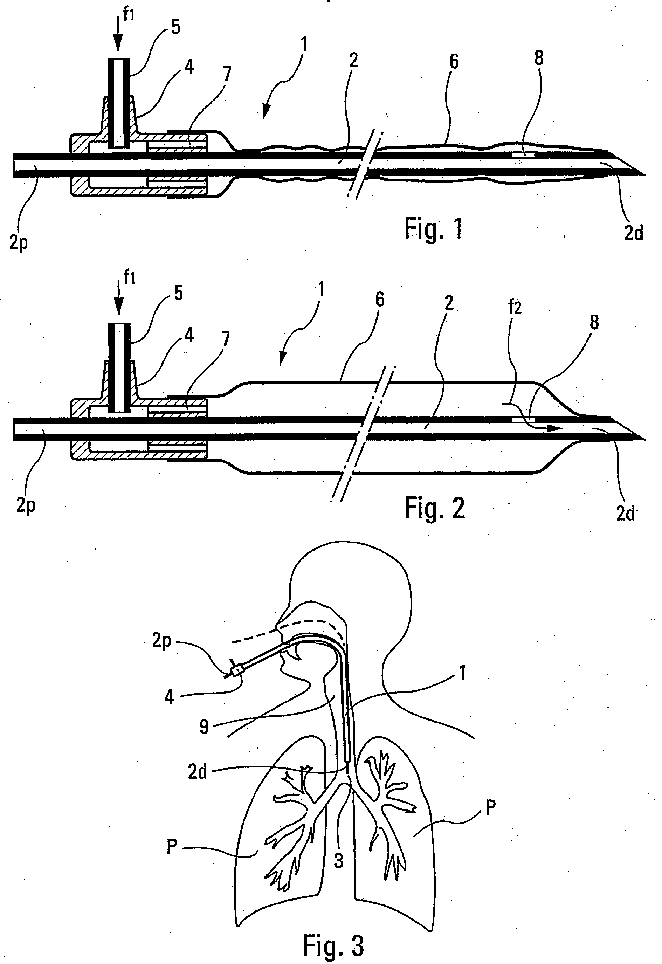

La sonde 1 conforme à la présente invention, montrée à titre d'exemple sur les figures 1 et 2, comporte un tube souple incompressible 2, par exemple réalisé en une matière synthétique telle qu'un chlorure de polyvinyle ou un polyéthylène, pourvu d'une extrémité distale 2d et d'une extrémité proximale 2p. L'extrémité distale 2d est destinée à être disposée dans la carène 3 d'un patient (voir la figure 3), alors que l'extrémité proximale 2p, extérieure audit patient, est reliée soit à l'air libre, soit à une source de gaz d'assistance respiratoire (non représentée).

L'extrémité proximale 2p du tube souple 2 est entourée par un embout 4, pourvu d'un conduit 5 apte à être relié à une source de gaz respiratoire de ventilation à basse pression (symbolisé par la flèche f1).

Une gaine souple gonflable 6, par exemple en un film plastique de quelques dizaines de micromètres, entoure le tube 2 sur la presque totalité de sa longueur et est reliée de façon étanche à ses deux extrémités, d'une part à l'embout 4 et, d'autre part, au tube 2 au voisinage de l'extrémité distale 2d.

A l'intérieur de l'embout 4, des canaux 7 mettent en communication le conduit 5 et l'intérieur de la gaine souple gonflable 6. par ailleurs, l'intérieur de ladite gaine souple gonflable 6 est mis en communication avec l'intérieur du tube 2 par au moins un trou 8 traversant la paroi latérale dudit tube et disposé du côté distal de ladite gaine.

La sonde 1 peut être introduite dans la trachée 9 du patient, soit par voie buccale (dessin en trait plein sur la figure 3), soit par voie nasale (pointillés sur la figure 3). Dans ce dernier cas, la sonde 1 peut comporter deux tubes 2 en parallèle à partir de l'embout 4 (un tube par narine). Lors de l'introduction, la gaine 6 est à l'état dégonflé (figure 1) et la longueur de la sonde 1 est telle que l'extrémité distale 2d atteigne la carène 3.

Une fois mise en place comme indiqué sur la figure 3, le conduit 5 est alimenté en gaz respiratoire de ventilation (flèche f1) de sorte que, d'une part, la gaine 6 se gonfle (figure 2), ce qui évite le contact des muqueuses du patient avec le tube 2, et que, d'autre part, du gaz respiratoire passe de la gaine 6 dans le tube 2 à travers les trous 8 (flèche f2). Il en résulte que du gaz respiratoire sort de l'extrémité distale 2d de la sonde 1, au niveau de la carène 3, pour alimenter les poumons P du patient sans espace mort, ni gaspillage.

L'expiration du patient se fait à travers le tube 2, de l'extrémité distale 2d vers l'extrémité proximale 2p.

Pour que ladite gaine souple 6 prenne, à l'état gonflé (figure 2) une forme prédéterminée, il est avantageux qu'elle soit préformée.

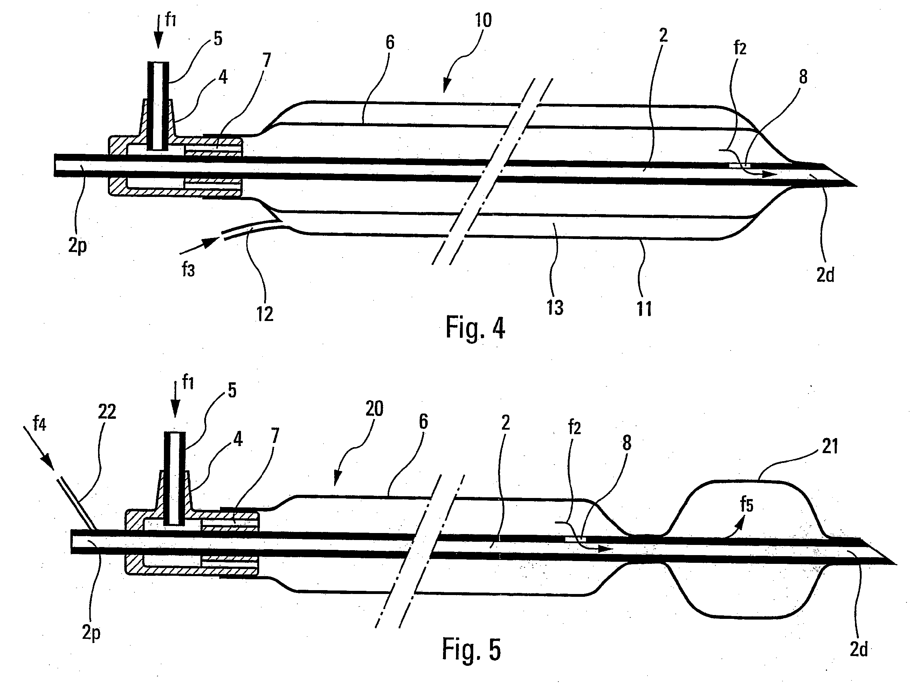

Dans les variantes de réalisation 10 et 20 illustrées respectivement par les figures 4 et 5, la sonde conforme à la présente invention comporte les éléments 2 et 4 à 8 décrits ci-dessus.

La sonde 10 de la figure 4 comporte de plus une gaine souple gonflable 11, semblable à la gaine 6, entourant cette dernière et reliée de façon étanche à ses deux extrémités, d'une part à l'embout 4 et, d'autre part, au tube 2 au voisinage de l'extrémité distale 2d. Du côté proximal, la gaine souple 11 est reliée à un conduit 12, par l'intermédiaire duquel on peut introduire un gaz de gonflage (symbolisé par la flèche f3) dans l'espace 13 compris entre les gaines 6 et 11. Ainsi, la sonde 10 est particulièrement confortable pour le patient puisqu'elle comporte deux coussins gazeux superposés (celui constitué entre le tube 2 et la gaine 6 et celui formé entre la gaine 6 et la gaine 11). De plus, la gaine 11 peut servir à assurer l'étanchéité entre la sonde 10, la carène 3 et la trachée 9, de façon à éviter que les sucs gastriques du patient passent dans les poumons P.

Dans la sonde 20 de la figure 4, la gaine 6 ne recouvre pas le tube 2 jusqu'à son extrémité distale 2d et cette dernière porte de façon connue un ballonnet gonflable 21. Celui-ci est relié -par exemple par l'intermédiaire d'un canal ménagé dans l'épaisseur de la paroi du tube 2 et non visible sur la figure 5- à un conduit 22 disposé du côté proximal de la sonde 20 et apte à être relié à une source de gaz de gonflage. Ainsi, le gaz traversant le conduit 22 (symbolisé par la flèche f4) peut déboucher dans le ballonnet 21 (flèche f5) pour gonfler ce dernier et ainsi assurer l'étanchéité entre la sonde 20 et la carène 3. Les poumons P du patient sont alors isolés des sucs gastriques.

## Revendications

1. Sonde respiratoire (1) comportant au moins un tube souple incompressible (2) ouvert à ses deux extrémités opposées, une extrémité proximale (2p) destinée à rester à l'extérieur d'un patient et une extrémité distale (2d) destinée à être disposée dans une trachée dudit patient, ledit tube étant apte à être introduit par voie buccale ou nasale dans la trachée (9) du patient, ladite sonde étant destinée à alimenter en gaz respiratoire ledit patient,
**caractérisée en ce que** ledit tube (2) est entouré, sur au moins une partie de sa longueur, par au moins une gaine de protection souple gonflable (6, 11) permettant d'isoler, dudit tube souple, les muqueuses des voies respiratoires du patient, ladite gaine de protection (6) étant reliée d'une façon étanche à ses deux extrémités, d'une part côté proximal destinée à rester à l'extérieur du patient, à un embout (4) entourant ledit tube (2) et, d'autre part côté distal au dit tube au voisinage de son extrémité distale (2d),
et **en ce que** le gaz respiratoire peut être introduit dans la gaine de protection (6) par l'embout afin d'être acheminé de l'extérieur du patient vers la trachée en étant conduit dans et par ladite gaine de protection (6), au moins un passage traversant (8) étant percé dans ledit tube souple incompressible (2), du côté distal de la sonde et à l'intérieur de ladite gaine de protection (6), afin que ledit gaz respiratoire puisse passer de ladite gaine de protection vers ledit tube souple (2) puis dans la trachée dudit patient, ledit gaz respiratoire acheminé permettant eh outre de gonfler ladite gaine de protection (6).

2. Sonde respiratoire selon la revendication 1, **caractérisée en ce que** sa longueur est telle que, lorsqu'elle est en place dans les voies respiratoires supérieures du patient, son extrémité distale (2d) se trouve dans la carène (3) de ce dernier.

3. Sonde respiratoire selon l'une des revendications 1 à 2, **caractérisée en ce que** ladite gaine de protection souple (6) est préformée pour prendre une forme gonflée déterminée.

4. Sonde respiratoire selon l'une des revendications 1 à 3, **caractérisée en ce qu'**elle comporte une gaine souple gonflable supplémentaire (11) entourant de façon étanche ladite gaine de protection souple gonflable (6).

5. Sonde respiratoire selon la revendication 4, **caractérisée en ce que** la gaine souple gonflable supplémentaire (11) est reliée du côté proximal à un conduit 12 par l'intermédiaire duquel on peut introduire un gaz de gonflage (f3).

6. Sonde respiratoire selon l'une des revendications 1 à 3, **caractérisée en ce que** ladite gaine de protection souple gonflable (6) dégage l'extrémité distale (2d) dudit tube souple incompressible (2) et **en ce que** ladite extrémité distale (2d) dudit tube (2) porte un ballonnet gonflable (21).

7. Sonde respiratoire selon la revendication 6, **caractérisée en ce que** le ballonnet gonflable (21) peut être gonflé (f4) par un gaz de gonflage par au moins l'intermédiaire d'un canal ménagé dans l'épaisseur de la paroi du tube (2).

8. Sonde respiratoire selon la revendication 7, **caractérisée en ce que** le canal ménagé dans l'épaisseur de la paroi du tube (2) est relié côté proximal à un conduit (22) disposé du côté proximal de la sonde (20), ledit conduit (22) étant destiné à être relié à une source dudit gaz de gonflage (f4).

9. Sonde respiratoire selon l'une des revendications précédentes, **caractérisée en ce que** le tube souple incompressible (2) est en chlorure de polyvinyle ou en polyéthylène et **en ce que** la gaine de protection (6) est un film plastique de quelques dizaines de micromètres d'épaisseur.

10. Sonde respiratoire selon l'une des revendications précédentes, **caractérisée en ce que** l'embout (4) est pourvu d'un conduit (5) apte à être relié à une source dudit gaz respiratoire (f1).

11. Sonde respiratoire selon la revendication 10, **caractérisée en ce que**, à l'intérieur de l'embout, des canaux (7) mettent en communication le conduit (5) et l'intérieur de la gaine souple gonflable (6).

## Patentansprüche

1. Beatmungstubus (1) mit wenigstens einem flexiblen, nicht zusammenpreßbaren, an dessen beiden entgegengesetzten Enden offenen Schlauch (2), wobei ein proximales Ende (2p) dazu bestimmt ist, außerhalb eines Patienten zu bleiben, und ein distales Ende (2d) dazu bestimmt ist, in einer Luftröhre des besagten Patienten angeordnet zu werden, wobei der Schlauch geeignet ist, durch den Mund oder durch die Nase in die Luftröhre (9) des Patienten eingeführt zu werden, wobei der Tubus dazu bestimmt ist, den Patienten mit einem Beatmungsgas zu versorgen,
**dadurch gekennzeichnet, daß** der Schlauch (2) auf wenigstens einem Teil seiner Länge von wenigstens einem flexiblen aufblasbaren Schutzschlauch (6, 11) umgeben ist, der es ermöglicht, die Schleimhäute der Luftwege des Patienten gegenüber dem flexiblen Schlauch zu isolieren, wobei der Schutzschlauch (6) an seinen beiden Enden dicht angeschlossen ist, und zwar zum einen - am proximalen Ende, das dazu bestimmt ist, außerhalb des Patienten zu verbleiben - an einem Anschlußstück (4), das den besagten Schlauch (2) umgibt, und zum anderen - am distalen Ende - am Schlauch im Bereich seines distalen Endes (2d), und
daß das Beatmungsgas durch das Anschlußstück in den Schutzschlauch (6) eingeleitet werden kann, um von außerhalb des Patienten her zur Luftröhre zugeführt zu werden, indem es in und durch den Schutzschlauch (6) geleitet wird, wobei in den flexiblen, nicht zusammenpreßbaren Schlauch (2) am distalen Ende des Tubus und im Inneren des Schutzschlauchs (6) ein Durchgangsloch (8) gebohrt ist, damit das Beatmungsgas vom Schutzschlauch in den flexiblen Schlauch (2) und dann in die Luftröhre des Patienten gelangen kann, wobei das zugeführte Beatmungsgas außerdem ermöglicht, den Schutzschlauch (6) aufzublasen.

2. Beatmungstubus gemäß Anspruch 1, **dadurch gekennzeichnet, daß** dessen Länge derart ist, daß sich dessen distales Ende (2d), wenn er in den oberen Luftwegen des Patienten angebracht ist, in der Luftröhrengabel (3) des letzteren befindet.

3. Beatmungstubus gemäß einem der Ansprüche 1 bis 2, **dadurch gekennzeichnet, daß** der flexible Schutzschlauch (6) vorgeformt ist, um aufgeblasen eine bestimmte Form anzunehmen.

4. Beatmungstubus gemäß einem der Ansprüche 1 bis 3, **dadurch gekennzeichnet, daß** er einen zusätzlichen flexiblen aufblasbaren Schlauch (11) aufweist, der den aufblasbaren flexiblen Schutzschlauch (6) dicht umhüllt.

5. Beatmungstubus gemäß Anspruch 4, **dadurch gekennzeichnet, daß** der zusätzliche flexible aufblasbare Schlauch (11) am proximalen Ende mit einem Rohr (12) verbunden ist, mittels dessen man ein Gas zum Aufblasen (f3) einleiten kann.

6. Beatmungstubus gemäß einem der Ansprüche 1 bis 3, **dadurch gekennzeichnet, daß** der aufblasbare flexible Schutzschlauch (6) das distale Ende (2d) des flexiblen, nicht zusammenpreßbaren Schlauchs (2) freiläßt und daß das distale Ende (2d) des besagten Schlauchs (2) einen aufblasbaren Ballon (21) aufweist.

7. Beatmungstubus gemäß Anspruch 6, **dadurch gekennzeichnet, daß** der aufblasbare Ballon (21) wenigstens über einen in der Dicke der Wandung des Tubus (2) angeordneten Kanal mit einem Gas zum Aufblasen aufgeblasen werden kann (f4).

8. Beatmungstubus gemäß Anspruch 7, **dadurch gekennzeichnet, daß** der in der Dicke der Wandung des Tubus (2) angeordnete Kanal am proximalen Ende an ein Rohr (22) angeschlossen ist, das am proximalen Ende des Tubus (20) angeordnet ist, wobei das Rohr (22) dazu bestimmt ist, an eine Quelle des Gases zum Aufblasen (f4) angeschlossen zu werden.

9. Beatmungstubus gemäß einem der vorangehenden Ansprüche, **dadurch gekennzeichnet, daß** der flexible, nicht zusammenpreßbare Schlauch (2) aus Polyvinylchlorid oder aus Polyäthylen ist und daß der Schutzschlauch (6) ein Plastikfilm mit einer Dicke von einigen Zehnfachen eines Mikrometers ist.

10. Beatmungstubus gemäß einem der vorangehenden Ansprüche, **dadurch gekennzeichnet, daß** das Anschlußstück (4) mit einem Rohr (5) versehen ist, das dazu geeignet ist, an eine Quelle des Beatmungsgases (f1) angeschlossen zu werden.

11. Beatmungstubus gemäß Anspruch 10, **dadurch gekennzeichnet, daß** im Inneren des Anschlußstücks Kanäle (7) das Rohr (5) mit dem Inneren des flexiblen aufblasbaren Schlauchs (6) verbinden.

## Claims

1. A respiratory probe (1) comprising at least one incompressible flexible tube (2) opened at its two opposed ends, a proximal end (2p) configured to stay external to a patient and a distal end (2d) configured to be arranged a trachea of said patient, said tube being configured to be introduced by the oral or nasal route into the trachea (9) of the patient, said probe being configured to supply with respiratory gas said patient,
**characterized in that** said tube (2) is surrounded, on at least a part of its length, by at least one inflatable flexible protective sheath (6, 11) for isolating the mucous membranes of the patient's airways from said flexible tube, said protective sheath (6) being connected in a leaktight manner at its two ends, on a first part, at the proximal side configured to stay external to the patient, to a connector piece (4) surrounding said tube (2) and, on the other part, at the distal side to said tube in the vicinity of its distal end (2d),
and **in that** the respiratory gas can be introduced in the protective sheath (6) through the connector piece in order to be sent from the exterior of the patient toward the trachea by being driven into and by said protective sheath (6), at least one through-passage (8) being pierced in said incompressible flexible tube (2), on the distal side of the probe and inside said protective sheath (6), in order that said respiratory gas may pass from said protective sheath toward said flexible tube (2) and then in the trachea of said patient, said sent respiratory gas further allowing to inflate said protective sheath (6).

2. The respiratory probe according to claim 1, **characterized in that** its length is such that, when it is in place in the patient's upper airways, its distal end (2d) is located in the carina (3) of the latter.

3. The respiratory probe according to one of claims 1 to 2, **characterized in that** said flexible protective sheath (6) is preformed in order to assume a defined inflated shape.

4. The respiratory probe according to one of claims 1 to 3, **characterized in that** it comprises an additional inflatable flexible sheath (11) that surrounds said inflatable flexible protective sheath (6) in a leaktight manner.

5. The respiratory probe according to claim 4, **characterized in that** the additional inflatable flexible sheath (11) is connected on the proximal side to a conduit (12) by which an inflation gas (f3) can be introduced.

6. The respiratory probe according to one of claims 1 to 3, **characterized in that** said inflatable flexible protective sheath (6) clears off the distal end (2d) of said incompressible flexible tube (2) and **in that** said distal end (2d) of said tube (2) carries an inflatable balloon (21).

7. The respiratory probe according to claim 6, **characterized in that** the inflatable balloon (21) may be inflated (f4) with an inflation gas by at least one channel formed in the thickness of the wall of the tube (2).

8. The respiratory probe according to claim 7, **characterized in that** the channel formed in the thickness of the wall of the tube (2) is connected at the proximal side to a conduit (22) arranged at the proximal side of the probe (20), said conduit (22) being able to be connected to a source of said inflation gas (f4).

9. The respiratory probe according to anyone of previous claims, **characterized in that** the incompressible flexible tube (2) is made of polyvinyl chloride or polyethylene and **in that** the protective sheath (6) is a plastic film of a few tens of micrometers thickness.

10. The respiratory probe according to anyone of previous claims, **characterized in that** the connector piece (4) is provided with a conduit (5) that can be connected to a source of said respiratory gas (f1).

11. The respiratory probe according to claim 10, **characterized in that**, inside the connector piece, channels (7) provide communication between the conduit (5) and the inside of the flexible protective sheath (6).
